# EUROPEAN PATENT SPECIFICATION

(11) **EP 1 614 677 B1**
(45) Date of publication and mention of the grant of the patent: **05.08.2009**
(21) Application number: 05104384.2
(22) Date of filing: 24.05.2005
(51) Int. Cl.: C07C 315/02, C07C 319/14, C07C 319/20

(54) **Improved procedure for the synthesis of bicalutamide**
Verbessertes Verfahren zur Herstellung von Bicalutamid
Procédé amélioré pour la synthèse de la bicalutamide

(30) Priority: 17.06.2004 IT MI20041222
(43) Date of publication of application: 11.01.2006
(73) Proprietor: Cosma S.p.A., 24040 Ciserano (IT)
(72) Inventor: Pizzatti, Enrica, 23020 Poggi Ridenti (IT); Vigano', Enrico, 22040 Lurago D'Erba (IT); Lussana, Massimiliano, 24020 Gorle (IT); Landonio, Ernesto, 20027 Rescaldina (IT)
(74) Representative: Appoloni, Romano

(56) References cited:
- EP-A- 0 100 172
- US-A- 4 636 505
- TUCKER H ET AL: "NONSTEROIDAL ANTIANDROGENS. SYNTHESIS AND STRUCTURE-ACTIVITY RELATIONSHIPS OF 3-SUBSTITUTED DERIVATIVES OF 2- HYDROXYPROPIONANILIDES" JOURNAL OF MEDICINAL CHEMISTRY, AMERICAN CHEMICAL SOCIETY. WASHINGTON, US, vol. 31, no. 5, 1 May 1988 (1988-05-01), pages 954-959, XP000605264 ISSN: 0022-2623
- TUCKER H ET AL: "Nonsteroidal Antiandrogens. Synthesis and Structure-Activity Relationships of 3-Substituted Derivatives of 2-Hydroxyproprionanilides" 1988, JOURNAL OF MEDICINAL CHEMISTRY, AMERICAN CHEMICAL SOCIETY. WASHINGTON, US, PAGE(S) 954-959 , XP002310037 ISSN: 0022-2623 schemes I and II

## Description

Bicalutamide, or N-[4-cyano-3-(trifluoromethyl)-phenyl-]-3-[4-fluorophenylsulfonyl]-2-hydroxy-2-methyl-propionamide, is a non steroidal anti androgen used in the treatment of prostate cancer (pharmaceutical speciality Casodex™). This active ingredient competes with testosterone and dihydrotestosterone in binding to receptor sites on the prostate and other androgen sensitive human tissues, with high activity and very few side effects.

This is described, along with preparative methods in EP 100172 (in the name ICI).

The following scheme shows the essential synthesis as described in the aforementioned EP 100172.

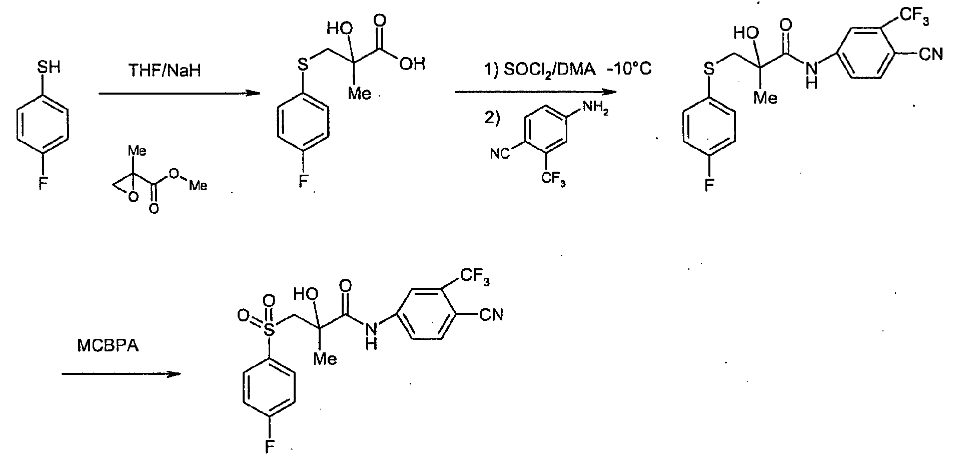

The main problem with this known synthesis is that its yield is too low, averaging about 50% (calculated relative to the first step, that is to say to obtain 2-hydroxy-2-methyl-3-(4-fluorophenylthio) propionic acid). Furthermore, the use of undesirable reagents, such as NaH, or operating conditions, for example the use of thionyl chloride at low temperatures, is required.

With respect to this known synthetic pathway, the present invention proposes an improved procedure for the synthesis of bicalutamide, consisting of the steps of
a) reaction between alkyl 2-methyl-oxirane-carboxylate and 4-fluorothiophenol to give 2-hydroxy-2-methyl-3-(4-fluorophenylthio) propionic acid,
b) reaction with 4-amino-2-trifluoromethyl-benzonitrile to give N-[4-cyano-3-(trifluoromethyl)-phenyl-]-3-[4-fluorophenylthio]-2-hydroxy-2-methyl propionamide,
c) oxidation of said N-[4-cyano-3-(trifluoromethyl)-phenyl-]-3-[4-fluorophenylthio]-2-hydroxy-2-methyl propionamide to give bicalutamide,
characterised in that said 2-hydroxy-2-methyl-3-(4-fluorophenylthio) propionic acid produced in step a) before undergoing step b) undergoes a step a') of acylation of the hydroxyl group in position 2 to give an intermediate 2-acyloxyl-2-methyl-3-(4-fluorophenylthio) propionic acid, which, in the course of step b) generates a successive intermediate N-[4-cyano-3-(trifluoromethyl)-phenyl-]-3-[4-fluorophenylthio]-2-acyloxy-2-methyl propionamide.

The procedure according to the invention surprisingly allows the achievement of a nearly quantitative yield.

The procedure which is the object of the invention is summarised in the following, non limiting, synthesis schemes, each relating to a different embodiment:

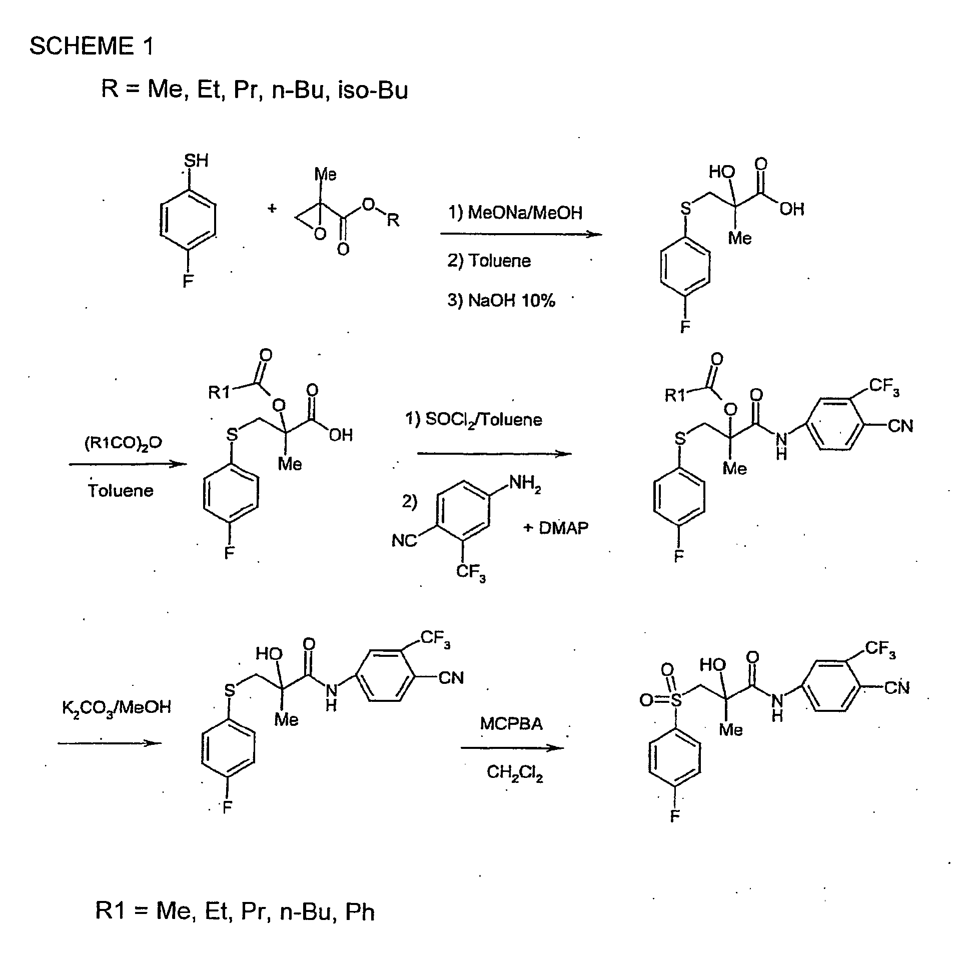

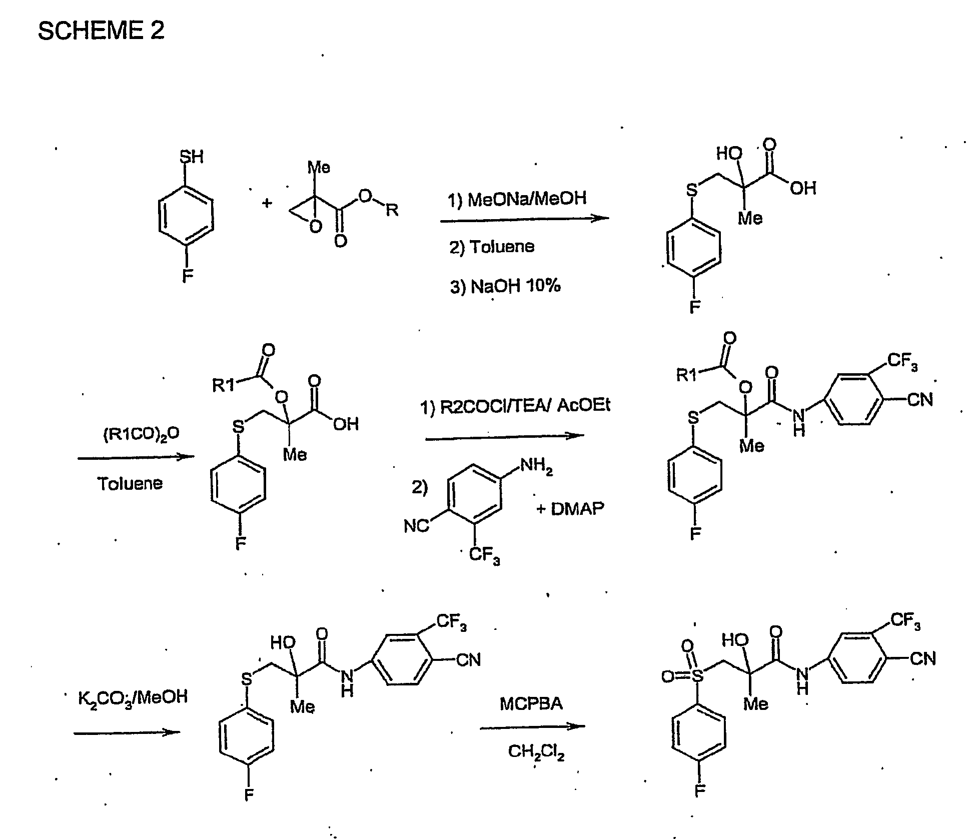

As indicated in the preceding schemes, the alkyl group R is preferably chosen from methyl, ethyl, propyl, butyl, isobutyl.
If butyl 2-methyl-oxirane-carboxylate is used, according to the invention this is preferably synthesised by oxidising butyl 2-methyl acrylate with m-chloroperbenzoic acid (MCPBA) according to the scheme:

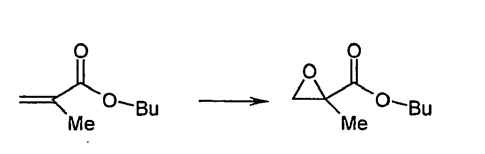

An analogous synthetic method can be used for R chosen from ethyl, propyl, isobutyl.

In the procedure according to the invention, in said step a') the acylation agent is the corresponding anhydride related to R1 in the schemes shown above, or where R1 is chosen from methyl, ethyl, propyl, butyl, phenyl.

In one embodiment, in said step a') said 2-hydroxy-2-methyl-3-(4-fluorophenylthio) propionic acid is dissolved in toluene.

In one embodiment of the procedure according to the invention, in said step a) said 4-fluorothiophenol is dissolved in anhydrous methanol, the solution is cooled and then a solution of methylate in methanol is added, the methanol is evaporated and the residue is taken up in anhydrous toluene and said alkyl 2-methyl-oxarane-carboxylate is added to this solution. The hydrolysis of the obtained alkyl ester is then carried out in a biphasic system of toluene/sodium hydroxide solution in water, for example a 10% solution.

In a different embodiment of the procedure according to the invention, in said step a) water is used as the solvent without the need in this way to isolate the thiophenolate or change solvent.

The reaction can be carried out in water with stoichiometric KHCO₃ or with NaOH, preferably in 5% excess over stoichiometric.

In another embodiment of the procedure according to the invention, in said step a) toluene is used as the solvent, with catalysis either by 3% of MeONa (used as a 30% solution) or by 3% of MeONa (used as a 95% solid).

In another embodiment of the procedure according to the invention, in said step a) the reagents are used without solvent, either in the absence of base or with catalysis by 3% of MeONa (used as a 30% solution).

In an embodiment of the procedure according to the invention as summarised in said Scheme 1, in said step b) thionyl chloride is added to a solution of said intermediate 2-acyloxy-2-methyl-3-(4-fluorophenylthio) propionic acid in anhydrous toluene to give the corresponding acid chloride.

In this embodiment of the procedure according to the invention, in said step b) to said acid chloride is added an organic base, chosen from 4-dimethylaminopyridine (DMAP), TEA, pyridine, picoline, lutidine, or inorganic, such as carbonate or bicarbonate, followed by 4-amino-2-trifluoromethyl-benzonitrile to give the intermediate N-[4-cyano-3-(trifluoromethyl)-phenyl-]-3-[4-fluorophenylthio]-2-acyloxy-2-methyl-propionamide. This last reaction is performed in a solvent chosen from aromatic and aliphatic hydrocarbons, ethers, ketones, esters, haloalkanes, with a maximum reaction temperature of 100°C. In a different embodiment this last reaction is performed in a solvent chosen from the polar aprotics; DMA, DMF, NMP, DMI, acetonitrile, with a maximum reaction temperature of 60°C.

In an alternative embodiment of the procedure according to the invention as summarised in said Scheme 2, in said step b) to a solution of said intermediate 2-acyloxy-2-methyl-3-(4-fluorophenylthio) propionic acid in a solvent chosen from the following: aromatic and aliphatic hydrocarbons, ethers, ketones, esters, haloalkanes, is added alkylchloroformate in the presence of an inorganic base (carbonate, bicarbonate) or a tertiary aliphatic or heteroaromatic organic base, preferably triethylamine (TEA), to give the corresponding mixed anhydride, to which 4-amino-2-trifluoromethyl-benzonitrile is subsequently added to give the intermediate N-[4-cyano-3-(trifluoromethyl)-phenyl-]-3-[4-fluorophenylthio]-2-acyloxy-2-methyl-propionamide. This last reaction is performed in a solvent chosen from aromatic and aliphatic hydrocarbons, ethers, ketones, esters, haloalkanes, and apolar protic solvents.

For the meaning of alkyl in said acyl chloride, see the list of substituents R₂ in Scheme 2.

In an embodiment of the procedure according to the invention, in said step b) said intermediate N-[4-cyano-3-(trifluoromethyl)-phenyl-]-3-[4-fluorophenylthio]-2-acyloxy-2-methyl-propionamide is hydrolysed to give said N-[4-cyano-3-(trifluoromethyl)-phenyl-]-3-[4-fluorophenylthio]-2-hydroxy-2-methyl-propionamide.

In an embodiment of the procedure according to the invention, in said step b) said hydrolysis is performed in methanol by the addition of potassium carbonate.

In an embodiment of the procedure according to the invention, in said step c) said N-[4-cyano-3-(trifluoromethyl)-phenyl-]-3-[4-fluorophenylthio]-2-hydroxy-2-methyl-propionamide dissolved in CH₂Cl₂ is reacted with m-chloroperbenzoic acid to give bicalutamide.

Said intermediates 2-acyloxy-2-methyl-3-(4-fluorophenylthio) propionic acid and N-[4-cyano-3-(trifluoromethyl)-phenyl-]-3-[4-fluorophenylthio]-2-acyloxy-2-methyl-propionamide are also objects of the invention.

In particular, said intermediates 2-acetoxy-2-methyl-3-(4-fluorophenylthio) propionic acid and N-[4-cyano-3-(trifluoromethyl)-phenyl-]-3-[4-fluorophenylthio]-2-acetoxy-2-methyl-propionamide are objects of the invention.

With the aim of better understanding the characteristics and the advantages of the invention, the following non limiting examples describe the practical implementation of the invention.

### Example 1

### Step a)

7.8 g (0.06 moles) of 4-fluorothiophenol are dissolved in 50 mL of anhydrous methanol and 11.12 g of a sodium methylate solution in methanol (0.0618 moles) is added with cooling; the mixture is stirred for 15 minutes and the solvent is then evaporated. The residue is taken up in anhydrous toluene and re-evaporated to ensure the complete elimination of any residual methanol.

The white solid is resuspended in 80 mL of anhydrous toluene with mechanical stirring and 7.15 g (0.061 moles) of methyl 2-methyl-oxirane-carboxylate is added maintaining the temperature between 20-25 °C. At the end of the addition the mixture is homogeneous. The solution is heated at 60 °C for 3 hours then cooled to 10 °C, it is then diluted with 50 mL of water and 15 mL of 36% NaOH is added. After being left overnight at r. t. (room temperature), the organic phase is separated and the aqueous phase is carefully acidified until pH 3 with 36% HCl.

After extracting twice with dichloromethane, the extracts are dried and the solvent evaporated under reduced pressure. The residue, 2-hydroxy-2-methyl-3-(4-fluorophenylthio) propionic acid, is treated with hot hexane and then filtered. The resulting yield is nearly quantitative.

TLC: toluene/dioxane/acetic acid 45/20/1.

Can be recrystallised from toluene/petroleum ether 5/1, m. pt. 95.5-97 °C.

### Example 2

### Step a')

To a solution of 2-hydroxy-2-methyl-3-(4-fluorophenylthio) propionic acid prepared in said example 1 (13.66 g, 0.0593 moles) in 70 mL of anhydrous toluene is added acetic anhydride (6.01 mL, 0.0623 moles) and the resulting solution is heated at 100 °C for 5h. After cooling to room temperature the solution is washed twice with 20 mL of water, the combined organic phases are dried and removed under reduced pressure. A dense oil is obtained which solidifies spontaneously. Single compound by TLC (eluent: dioxane/toluene/acetic acid: 45/20/1). Quantitative yield.

### Example 3

### Step b)

To a solution of the acetoxy derivative prepared in said example 2 (4.0 g, 0.0146 moles) in 20 mL of anhydrous toluene at r. t. is added 1.27 mL (0.0148 moles) of thionyl chloride and then heated at 85-90 °C (external temperature) for 3-4 h. The solvent is completely evaporated under reduced pressure. The product is obtained as an oil which can be used as is in the successive reaction.

### Example 4

### Step b)

To a solution of the acid chloride as prepared in the previous example (2.1 g, 0.0072 moles) in 12 mL of anhydrous toluene is added 0.9 g (0.0072 moles) of DMAP, maintaining the solution at room temperature; the suspension is allowed to react for 10 minutes then 1.15 g (0.0062 moles) of 4-amino-2-trifluoromethylbenzonitrile dissolved in toluene is added and the mixture is heated at 75-80 °C (external temperature) for 8-10 hours following the disappearance of the amine by TLC (eluent: ethyl acetate/ toluene 75/25 or CH₂Cl₂). After cooling to room temperature the solution is diluted with toluene and treated with a 5% solution of HCl, separated and washed with 5% bicarbonate solution. The organic phase is dried and evaporated under reduced pressure. Crude N-[4-cyano-3-(trifluoromethyl)-phenyl-]-3-[4-fluorophenylthio]-2-acetoxy-2-methyl-propionamide is obtained in quantitative yield as shown by HPLC analysis, NMR spectroscopy and TLC.

### Example 5

### Step b)

The reaction is performed using the same method as described in example 4, but using DMF as the solvent and heating at 45-50 °C (internal temperature). The reaction time is slightly longer than that of the reaction in toluene reported above in example 4.

### Example 6

### Step b)

To a solution of the acetoxy derivative prepared in said example 2 (4.0 g, 0.0146 moles) in 20 mL of anhydrous toluene at room temperature is added 2.23 mL (0.016 moles) of triethylamine. After cooling to 0 °C, 1.46 mL ( 0.0153 moles) of ethyl chloroformate is slowly added. After 1 hour the precipitated triethylamine hydrochloride formed is filtered off and 2.60 g (0.0140 moles) of 4-amino-2-trifluromethyl-benzonitrile dissolved in toluene is added to the filtrate and the resulting solution is heated at 75-80 °C until disappearance of the reagents is observed by HPLC.

After cooling to room temperature the solution is washed with water, the phases are separated and dried with sodium sulphate and the solvent removed under reduced pressure. 5.85 g of crude intermediate is obtained which is used in the following reaction without further purification.

### Example 7

### Step b)

The amide formed as described in step 4, 5 or 6 is dissolved in methanol, and to it is added a 25% solution of potassium carbonate (1.1 moles per mole of material), the solution is then stirred at room temperature for 12 hours. Then the hydrolysis is complete. The methanol is evaporated and the residue taken up in water and extracted with dichloromethane. The mixture containing N-[4-cyano-3-(trifluoromethyl)-phenyl-]-3-[4-fluorophenylthio]-2-hydroxy-2-methyl-propionamide can be used as is in the following reaction.

An alternative extraction solvent is ethyl acetate. The product can be further purified by treating the ethyl acetate solution with activated carbon and, once the solvent is dried, recrystallising from toluene.

### Example 8

### Step c)

To a solution of the amide prepared according to example 7 (5.67 g, 0.0142 moles) in 70 ml of CH₂Cl₂ is added, portion wise, 7.37 g of 75% m-chloroperbenzoic acid (0.0299 moles) maintaining the temperature at 25 °C and the solution is stirred for 5 hours. The reaction is slightly exothermic (in particular during the addition of the first of the two equivalents) and before the second equivalent is added a precipitate begins to form which increases with time. After further dilution, a solution of 5% sodium carbonate is added and the mixture stirred for 1 hour, the phases are then separated and the solvent evaporated under reduced pressure. The solid obtained is purified with diisopropyl ether. Quantitative yield with general absence of sulfoxide, small amounts of uncharacterised impurities are present.

Examples of variations in the execution of step a) are described below. In these examples 2-methyl-oxirane-methylcarboxylate is also called methyl 2-methylglycidate.

### Example 9

### Step a)

4-Fluorothiophenol (93.6 g, m.w.: 128.17, 0.73 moles) is placed in a round bottomed flask with H₂O (500 cc), KHCO₃ (73.5 g, m.w.: 100.1, 0.734 moles) and methyl 2-methylglycidate (85.7 g, m.w.: 116.1, 0.738 moles) and the temperature is maintained at 50-55 °C for 3.5 hours.

TLC shows that the reaction is complete, with major presence of the intermediate methyl ester, and only slight traces of other products.

30% NaOH is added (221 g, m.w.: 40, 1.65 moles) and the mixture is heated at 60 °C for 30 minutes.

TLC shows that the reaction is complete.

The solution is cooled to 20-25 °C, acidified to pH 2.5 with 36% HCl, stirred for 30 minutes and the product is then filtered off, washing with water (480 cc) and then with heptane (240 cc).

Drying under vacuum gives 155.85 g of a white solid (theoretical 168.1 g) yield = 92.7% dt
Tit (NaOH) = 98.4%, K.F. = 0.01%

To confirm that the transformation is quantitative, quantification of the mother liquor (by TLC) yields a further 8 g of product (bringing the yield to 97.5% dt).

### Example 10

### Step a)

4-Fluorothiophenol (37.44 g, m.w.: 128.17, 0.292 moles) is placed in a round bottomed flask with H₂O (80 cc) and 30% NaOH (40.9 g, m.w.: 40, 0.307 moles), methyl 2-methylglycidate (34.6 g. m.w.: 116.1, 0.298 moles) is then added slowly over an hour at 25 °C, after a further 30 minutes, TLC shows that the reaction is complete.

30% NaOH is added (2 g) and the mixture is heated at 60 °C for 60 minutes.

TLC shows practically only the spot of the desired 2-hydroxy-2-methyl-3-(4-fluorophenylthio) propionic acid.

The solution is cooled to 20-25 °C, acidified to pH 2 with 36% HCl, diluted with water (250 mL) and stirred for 30 minutes, the product is filtered off and washed with water (100 cc) until no chloride remains.

Drying under vacuum gives 63.1 g of a white solid (theoretical 67.2 g) yield = 93.8% dt
Tit (NaOH) = 98.35%, TLC: single spot, HPLC = 100%

To confirm that the transformation is nearly quantitative, quantification of the mother liquor (by TLC) yields a further 2.6 g of product (bringing the yield to 97.7% dt).

### Example 11

### Step a)

4-Fluorothiophenol (37.44 g, m.w.: 128.17, 0.292 moles) is placed in a round bottomed flask with toluene (80 cc) and MeONa (30% solution in MeOH) (1.6 g, m.w.: 54, 0.0089 moles), methyl 2-methylglycidate (34.6 g. m.w.: 116.1, 0.298 moles) is then added slowly over an hour at 35-40 °C, after a further 30 minutes, TLC shows that the reaction is complete, with major presence of the methyl ester intermediate.

Water (80 cc) is added, followed by 30% NaOH solution (42.8 g, m.w. 40, 0.321 moles) and the mixture is heated at 60 °C for 30 minutes.

TLC shows practically only the spot of the desired 2-hydroxy-2-methyl-3-(4-fluorophenylthio) propionic acid.

The solution is cooled to 20-25 °C, the toluene phase is removed, the aqueous phase is diluted with water (270 cc), acidified to pH 2 with 36% HCl and stirred for 30 minutes, the product is filtered off and washed with water (150 cc) until no chloride remains.

Drying under vacuum gives 58.9 g of a white solid (theoretical 67.2 g), yield = 87.6% dt
Tit (NaOH) = 99.69%, TLC: single spot, HPLC = 99.1 %

To confirm that the transformation is nearly quantitative, quantification of the mother liquor (by TLC) yields a further 6.6 g of product (bringing the yield to 97.5% dt).

### Example 12

### Step a)

4-Fluorothiophenol (22.83 g, m.w.: 128.17, 0.178 moles) is placed in a round bottomed flask and heated to 55-60 °C, methyl 2-methylglycidate (21.1 g. m.w.: 116.1, 0.181 moles) is then added slowly over an hour at 55-60 °C, after a further 60 minutes at 55-60 °C, TLC shows that the reaction is complete, with major presence of the methyl ester intermediate.

Water (213 cc) is added, followed by 30% NaOH solution (26.1 g, m.w.: 40, 0.195 moles) and the mixture is heated at 60 °C for 2 hours.

TLC shows only the spot of the desired 2-hydroxy-2-methyl-3-(4-fluorophenylthio) propionic acid.

The solution is cooled to 20-25 °C, acidified to pH 2 with 36% HCl and stirred for 30 minutes, the product is filtered off and washed with water (150 cc) until no chloride remains.

Drying under vacuum gives 35.16 g (theoretical 41 g), yield = 85.7% dt
Tit (NaOH) = 101%, TLC: single spot, HPLC = 99.8%

To confirm that the transformation is nearly quantitative, quantification of the mother liquor (by TLC) yields almost a further 6 g of product (bringing the yield to practically 100%).

### Example 13

### Step a)

4-Fluorothiophenol (37.44 g, m.w. : 128.17, 0.292 moles) is placed in a round bottomed flask with MeONa (30% solution in MeOH) (1.6 g, m.w.: 54, 0.0089 moles) and heated to 35-40 °C, methyl 2-methylglycidate (34.6 g. m.w.: 116.1, 0.298 moles) is then added over 15-30 minutes at 35-40 °C, after a further 30 minutes, TLC shows that the reaction is complete.

Water (110 cc) is added, followed by 30% NaOH solution (58.4 g, m.w.: 40, 0.438 moles) and the mixture is heated at 60 °C for 60 minutes.

TLC shows practically only the spot of the desired 2-hydroxy-2-methyl-3-(4-fluorophenylthio) propionic acid.

The solution is cooled to 20-25 °C, acidified to pH 2 with 36% HCl and stirred for 30 minutes, the product is filtered off and washed with water (350 cc) until no chloride remains.

Drying under vacuum gives 60.6 g of a white solid (theoretical 67.2 g), yield = 90.1 % dt
Tit (NaOH) = 100.03%, TLC: single spot, HPLC = 99.6%

To confirm that the transformation is nearly quantitative, quantification of the mother liquor (by TLC) yields a further 5 g of product (bringing the yield to 97.6% dt).

### Example 14

### Step a)

4-Fluorothiophenol (5.57 g, m.w.: 128.17, 0.043 moles) is placed in a round bottomed flask with water (11.9 mL) and KHCO₃ (0.85 g, m.w.: 100.1, 0.0085 moles) and heated to 35-40 °C, butyl 2-methylglycidate (7 g. m.w.: 158.18, 0.044 moles) is then added for 30 minutes at 35-40 °C and this temperature is maintained for 1 hour.

TLC shows that the reaction is complete, with major presence of the intermediate butyl ester.

30% NaOH is added (7.9 g, m.w.: 40, 0.059 moles) and the mixture is heated at 60 °C for 30 minutes.

TLC shows the complete disappearance of the butyl ester intermediate and the appearance of the required 2-hydroxy-2-methyl-3-(4-fluorophenylthio) propionic acid as practically a single spot

The butyl alcohol is removed under vacuum, the solution is acidified to pH 2.5 with 36% HCl, stirred for 30 minutes and the product is then filtered off and washed with enough water until no chloride remains.

Drying under vacuum gives 8.35 g of a white solid (theoretical 10.19 g), yield = 82% dt
Tit (NaOH) = 99.71%, TLC: single spot, HPLC = 99.6%

To confirm the utility of the transformation, quantification of the mother liquor (by TLC) yields a further 0.8 g of product (bringing the yield to at least 89.8% dt).

An example of a complete synthesis according to the invention is described below.

### Example 15

Step a): Reaction in water between 4-fluorothiophenol and methyl 2-methyl glycidate in the presence of potassium bicarbonate to give the intermediate methyl 2-hydroxy-2-methyl-3-(4-fluorophenylthio) propionate ("methyl ester").

Successive hydrolysis of the intermediate with 30% sodium hydroxide solution, and acidification of the reaction mixture to give 2-hydroxy-2-methyl-3-(4-fluorophenylthio) propionic acid.

Extraction of this intermediate "hydroxyacid" with toluene, and use of this solution in the following step.

Yield = 97% dt (based on 4-FT).
Step a'): reaction in toluene between 2-hydroxy-2-methyl-3-(4-fluorophenylthio) propionic acid and acetic anhydride (in the presence of catalytic 4-dimethylamino pyridine) to give 2-acetoxy-2-methyl-3-(4-fluorophenylthio) propionic acid (intermediate "acetylacid").
Use of this toluene solution in the following step.
Yield = 90% dt (based on 4-FT).
Step b): reaction in toluene between 2-acetoxy-2-methyl-3-(4-fluorophenylthio) propionic acid (intermediate "acetylacid") and thionyl chloride to give the intermediate 2-acetoxy-2-methyl-3-(4-fluorophenylthio) propionyl chloride (intermediate "chloride"). The successive reaction of this intermediate (still in toluene) with 4-cyano-3-trifluoromethyl aniline (in the presence of 4-dimethylamino pyridine) to give N-[4-cyano-3-(trifluoromethyl)-phenyl-]-3-[4-fluorophenylthio]-2-acetoxy-2-methyl-propionamide (intermediate "acetylsulfide"). Evaporation of the toluene and use of the residual oil in the following step.
Step b'): reaction in methanol/water between N-[4-cyano-3-(trifluoromethyl)-phenyl-]-3-[4-fluorophenylthio]-2-acetoxy-2-methyl-propionamide ("acetylsulfide") and potassium carbonate to give N-[4-cyano-3-(trifluoromethyl)-phenyl-]-3-[4-fluorophenylthio]-2-hydroxy-2-methyl-propionamide ("sulfide"). Isolation of this intermediate by centrifugation. Crystallisation of the crude from toluene. Isolation of this intermediate by centrifugation.

| | |
|---|---|
| Yield = 65.4 % dt | (based on aniline) |
| Yield = 139.9 % dp | (based on aniline) |
| Yield = 186.8 % dp | (based on 4-FT) |

Step c): reaction in dichloromethane between N-[4-cyano-3-(trifluoromethyl)-phenyl-]-3-[4-fluorophenylthio]-2-hydroxy-2-methyl-propionamide ("sulfide") and 3-chloroperbenzoic acid ("MCPBA") to give crude bicalutamide. Isolation of this intermediate by centrifugation from biphasic dichloromethane and water. Yield = 90% dt (based on dry crystallised sulphide)
Yield = 98% dp (based on dry crystallised sulphide)
Crystallisation of crude bicalutamide from 90% aqueous isopropyl alcohol to give crystalline bicalutamide. Isolation of the product by centrifugation and desiccation.
Yield = 90%.

Alternative recrystallisation solvents to isopropanol are isobutanol, 1-methoxy-2-propanol, ethyl acetate, MIBK or acetonitrile (preferably aqueous).

As can be seen from the complete description given above, with respect to the previously cited technique, the invention principally allows the advantages of a considerably improved yield, and the avoidance of the use of reagents, such as NaH, or operating conditions, for example in the case of the reaction with thionyl chloride at low temperature, which are dangerous and undesirable.

Furthermore, regarding step a), it resulted an ample variation in the choice of base and solvent (also neat), without the need to isolate the thiophenate. Using water as the solvent in step a), the acid is arrived at in a one-pot type reaction system.

According to the invention, there is the possibility of separating the two steps avoiding the isolation of the ester and expensive changes of solvent.

Another relevant advantage in step a) is the possibility of using a base in catalytic quantities.

## Claims

1. Process for the synthesis of bicalutamide (i.e. N-[4-cyano-3- (trifluoro methyl) -phenyl-]-3-[4-fluoro phenyl sulfonyl]-2-hydroxy- -2- methyl propionamide consisting of the steps of
a) reaction between alkyl 2-methyl-oxirome-carboxylate and 4-fluorothiophenol to give 2-hydroxy-2-methyl-3-(4-fluorophenylthio) propionic acid,
b) reaction with 4-amino-2-trifluoromethyl-benzonitrile to give N-[4-cyano-3-(trifluoromethyl)-phenyl-]-3-[4-fluorophenylthio]-2-hydroxy-2-methyl propionamide,
c) oxidation of the said N-[4-cyano-3-(trifluoromethyl)-phenyl-]-3-[4-fluorophenylthio]-2-hydroxy-2-methyl propionamide to give bicalutamide
**characterised in that** the said 2-hydroxy-2-methyl-3-(4-fluorophenylthio) propionic acid produced in step a) before undergoing said step b) undergoes a step a') of acylation of the hydroxyl group in position 2 to give an intermediate 2-acyloxyl-2-methyl-3-(4-fluorophenylthio) propionic acid, which, in the course of step b) generates a successive intermediate N-[4-cyano-3-(trifluoromethyl)-phenyl-]-3-[4-fluorophenylthio]-2-acyloxy-2-methyl propionamide.

2. Process according to claim 1, **characterised in that** in said step a') the acylation agent is an anhydride (R1CO)₂O where R1 is chosen from methyl, ethyl, propyl, n-butyl, phenyl.

3. Process according to claim 2, **characterised in that** in said step a') the acylation agent is acetic anhydride.

4. Process according to claim 1, **characterised in that** in said step a) said 4-fluorothiophenol is dissolved in anhydrous methanol, a solution of sodium methylate in methanol is added, the methanol is evaporated, the residue is taken up in anhydrous toluene, said alkyl 2-methyl-oxirane-carboxylate is added to the solution and then the obtained alkyl ester is hydrolysed in a biphasic system of toluene/sodium hydroxide in water.

5. Process according to claim 1, **characterised in that** in said step a) water is used as the solvent in the presence of carbonate or bicarbonate as a base.

6. Process according to claim 1, **characterised in that** in said step a) water is used as the solvent in the presence of alkali such as soda as a base.

7. Process according to claim 1, **characterised in that** in said step a) toluene is used as the solvent in the presence of sodium methylate as a base.

8. Process according to claim 1, **characterised in that** in said step a) alkyl 2-methyl-oxirane carboxylate and 4-fluorothiophenol are used without adding any solvent

9. Process according to claim 8, **characterised in that** in said step a) alkyl 2-methyl-oxirane-carboxylate and 4-fluorothiophenol are used in the presence of sodium methylate as a base

10. Process according to claims 5, 6, 7, 9, **characterised in that** said base is in catalytic quantity.

11. Process according to claim 1, **characterised in that** in said step a) alkyl 2-methyl-oxirane-carboxylate, in which the alkyl group is Et, Pr, n-Bu, iso-Bu, is prepared by the oxidation of the corresponding alkyl 2-methylacrylate with MCPBA.

12. Process according to claim 1, **characterised in that** in said step a) water with potassium bicarbonate is used as the solvent for alkyl 2-methyl-oxirane carboxylate.

13. Process according to claim 1, **characterised in that** in said step b) thionyl chloride is added to a solution of said intermediate 2-acyloxyl-2-methyl-3-(4-fluorophenylthio) propionic acid in anhydrous toluene to give the corresponding acid chloride.

14. Process according to claim 13, **characterised in that** in said step b) to said acid chloride is added an organic or inorganic base, followed by 4-amino-2-trifluoromethyl-benzonitrile to give the intermediate N-[4-cyano-3-(trifluoromethyl)-phenyl-]-3-[4-fluorophenylthio]-2-acyloxy-2-methyl propionamide.

15. Process according to claim 14, **characterised in that** in said step b) to said acid chloride is added an organic or inorganic base, followed by 4-amino-2-trifluoromethyl-benzonitrile to give the intermediate N-[4-cyano-3-(trifluoromethyl)-phenyl-]-3-[4-fluorophenylthio]-2-acyloxy-2-methyl propionamide in a solvent chosen from aromatic and aliphatic hydrocarbons, ethers, ketones, esters, haloalkanes, with a maximum reaction temperature of 100 °C.

16. Process according to claim 14, **characterised in that** in said step b) to said acid chloride is added an organic or inorganic base, followed by 4-amino-2-trifluoromethyl-benzonitrile to give the intermediate N-[4-cyano-3-(trifluoromethyl)-phenyl-]-3-[4-fluorophenylthio]-2-acyloxy-2-methyl propionamide in a solvent chosen from the polar aprotics: DMA, DMF, NMP, DMI, acetonitrile with a maximum reaction temperature of 100 °C.

17. Process according to claim 14, **characterised in that** said organic base is chosen from 4-dimethylaminopyridine (DMAP), pyridine, picoline or lutidine.

18. Process according to claim 1, **characterised in that** in said step b) to a solution of said intermediate 2-acyloxy-2-methyl-3-(4-flurophenylthio)-propionic acid was added acylchloride in the presence of a base, to give the corresponding mixed anhydride, to which 4-amino-2-trifluoromethyl-benzonitrile is successively added to give the intermediate N-[4-cyano-3-(trifluoromethyl)-phenyl-]-3-[4-fluorophenylthio]-2-acyloxy-2-methyl-propionamide.

19. Process according to claim 1, 14 and 18, **characterised in that** in said step b) said intermediate N-[4-cyano-3-(trifluoromethyl)-phenyl-]-3-[4-fluorophenylthio]-2-acyloxy-2-methyl-propionamide is hydrolysed to give said N-[4-cyano-3-(trifluoromethyl)-phenyl-]-3-[4-fluorophenylthio]-2-hydroxy-2-methyl-propionamide.

20. Process according to claim 19, **characterised by** the by the fact that in said step b) said hydrolysis is performed in methanol by the addition of potassium carbonate.

21. Process according to claim 1, **characterised in that** in said step a') and b) the solvent is toluene and the reaction system is one pot.

22. Process according to claim 1, **characterised in that** in said step c) said N-[4-cyano-3-(trifluoromethyl)-phenyl-]-3-[4-fluorophenylthio]-2-hydroxy-2-methyl-propionamide dissolved in CH₂Cl₂ is reacted with m-chloroperbenzoic acid to give bicalutamide.

23. Process according to claim 1, **characterised in that** a recrystallisation of crude bicalutamide to give crystallised Bicalutamide from a crystallisation solvent chosen from ethyl acetate, methyl isobutyl ketone MIBK, acetonitrile, isopropyl alcohol, isobutyl alcohol, or 1-methoxy-2-propanol, possibly aqueous.

24. Intermediate 2-acyloxy-2-methyl-3-(4-fluorophenylthio) propionic acid, in which said acyloxy group is chosen from R1 = methyl, ethyl, propyl, butyl, or phenyl.

25. Intermediate according to claim 24, wherein said acyloxy group is acetoxy.

26. Intermediate N-[4-cyano-3-(trifluoromethyl)-phenyl-]-3-[4-fluorophenylthio]-2-acyloxy-2-methyl-propionamide, in which said acyloxy group is chosen from R1 = methyl, ethyl, propyl, butyl, or phenyl.

27. Intermediate according to claim 26, wherein said acyloxy group is acetoxy.

## Patentansprüche

1. Verfahren zur Herstellung von Bicalutamid (d. h. N-[4-Cyano-3-(trifluormethyl)-phenyl]-3-[4-fluorphenylsulfonyl]-2-hydroxy-2-methylpropio namid), bestehend aus den folgenden Schritten:
a) Reaktion zwischen einem 2-Methyloxirancarbonsäurealkylester und 4-Fluorthiophenol, um 2-Hydroxy-2-methyl-3-(4-fluorphenylthio)propionsäure zu ergeben,
b) Reaktion mit 4-Amino-2-trifluormethylbenzonitril, um N-[4-Cyano-3-(trifluormethyl)-phenyl]-3-[4-fluorphenylthio]-2-hydroxy-2-methylpropionamid zu ergeben,
c) Oxidation des genannten N-[4-Cyano-3-(trifluormethyl)-phenyl]-3-[4-fluorphenylthio]-2-hydroxy-2-methylpropionami d, um Bicalutamid zu ergeben;
**dadurch gekennzeichnet, dass** die in Schritt a) hergestellte genannte 2-Hydroxy-2-methyl-3-(4-fluorphenylthio)propionsäure vor dem Einsetzen in Schritt b) einem Schritt a') der Acylierung der Hydroxylgruppe in Position 2 unterzogen wird, um ein Intermediat 2-Acyloxyl-2-methyl-3-(4-fluorphenylthio)propionsäure zu ergeben, aus welchem im Verlauf von Schritt b) ein nachfolgendes Intermediat N-[4-Cyano-3-(trifluormethyl)-phenyl]-3-[4-fluorphenylthio]-2-acyloxy-2-methylpropionami d entsteht.

2. Verfahren gemäß Anspruch 1, **dadurch gekennzeichnet, dass** in genanntem Schritt a') das Acylierungsmittel ein Anhydrid (R1CO)₂O ist, wobei R1 ausgewählt wird aus Methyl, Ethyl, Propyl, n-Butyl, Phenyl.

3. Verfahren gemäß Anspruch 2, **dadurch gekennzeichnet, dass** in genanntem Schritt a') das Acylierungsmittel Essigsäureanhydrid ist.

4. Verfahren gemäß Anspruch 1, **dadurch gekennzeichnet, dass** in genanntem Schritt a) genanntes 4-Fluorthiophenol in wasserfreiem Methanol gelöst wird, eine Lösung von Natriummethylat in Methanol zugegeben wird, Methanol abgedampft wird, der Rest in wasserfreiem Toluol aufgenommen wird, genannter 2-Methyloxirancarbonsäurealkylester zu der Lösung gegeben wird und anschließend der erhaltene Alkylester in einem zweiphasigen System aus Toluol/Natriumhydroxid in Wasser hydrolysiert wird.

5. Verfahren gemäß Anspruch 1, **dadurch gekennzeichnet, dass** in genanntem Schritt a) Wasser als Lösungsmittel in der Gegenwart von Carbonat oder Hydrogencarbonat als Base verwendet wird.

6. Verfahren gemäß Anspruch 1, **dadurch gekennzeichnet, dass** in genanntem Schritt a) Wasser als das Lösungsmittel in der Gegenwart einer Alkalimetallverbindung wie z. B. Natriumcarbonat als Base verwendet wird.

7. Verfahren gemäß Anspruch 1, **dadurch gekennzeichnet, dass** in genanntem Schritt a) Toluol als das Lösungsmittel in der Gegenwart von Natriummethylat als Base verwendet wird.

8. Verfahren gemäß Anspruch 1, **dadurch gekennzeichnet, dass** in genanntem Schritt a) 2-Methyloxirancarbonsäurealkylester und 4-Fluorthiophenol ohne Zugabe eines Lösungsmittels verwendet werden.

9. Verfahren gemäß Anspruch 8, **dadurch gekennzeichnet, dass** in genanntem Schritt a) 2-Methyloxirancarbonsäurealkylester und 4-Fluorthiophenol in der Gegenwart von Natriummethylat als Base verwendet werden.

10. Verfahren gemäß den Ansprüchen 5, 6, 7, 9, **dadurch gekennzeichnet, dass** die genannte Base in katalytischen Mengen vorliegt.

11. Verfahren gemäß Anspruch 1, **dadurch gekennzeichnet, dass** in genanntem Schritt a) 2-Methyloxirancarbonsäurealkylester, in der die Alkylgruppe Et, Pr, n-Bu oder iso-Bu ist, durch Oxidation des korrespondierenden 2-Methylacrylsäurealkylesters mit MCPBA hergestellt wird.

12. Verfahren gemäß Anspruch 1, **dadurch gekennzeichnet, dass** in genanntem Schritt a) Wasser mit Kaliumhydrogencarbonat als das Lösungsmittel für 2-Methyloxirancarbonsäurealkylester verwendet wird.

13. Verfahren gemäß Anspruch 1, **dadurch gekennzeichnet, dass** in genanntem Schritt b) Thionylchlorid zu einer Lösung des genannten Intermediats 2-Acyloxyl-2-methyl-3-(4-fluorphenylthio)propionsäure in wasserfreiem Toluol gegeben wird, um das entsprechende Säurechlorid zu erhalten.

14. Verfahren gemäß Anspruch 13, **dadurch gekennzeichnet, dass** in genanntem Schritt b) zu dem genannten Säurechlorid eine organische oder anorganische Base gegeben wird, gefolgt von 4-Amino-2-trifluormethylbenzonitril, um das Intermediat N-[4-Cyano-3-(trifluormethyl)-phenyl]-3-[4-fluorphenylthio]-2-acyloxy-2-methylpropionami d zu ergeben.

15. Verfahren gemäß Anspruch 14, **dadurch gekennzeichnet, dass** in genanntem Schritt b) zu diesem Säurechlorid eine organische oder anorganische Base gefolgt von 4-Amino-2-trifluormethylbenzonitril gegeben wird, um in einem Lösungsmittel ausgewählt aus aromatischen und aliphatischen Kohlenwasserstoffen, Ethern, Ketonen, Estern, Halogenalkanen, bei einer maximalen Reaktionstemperatur von 100 °C das Intermediat N-[4-Cyano-3-(trifluormethyl)-phenyl]-3-[4-fluorphenylthio]-2-acyloxy-2-methylpropionami d zu ergeben.

16. Verfahren gemäß Anspruch 14, **dadurch gekennzeichnet, dass** in genanntem Schritt b) zu dem genannten Säurechlorid eine organische oder anorganische Base gefolgt von 4-Amino-2-trifluormethylbenzonitril gegeben wird, um in einem Lösungsmittel ausgewählt aus den polaren aprotischen Lösungsmitteln DMA, DMF, NMP, DMI oder Acetonitril bei einer maximalen Reaktionstemperatur von 100°C das Intermediat N-[4-Cyano-3-(trifluormethyl)-phenyl]-3-[4-fluorphenylthio]-2-acyloxy-2-methylpropionami d zu ergeben.

17. Verfahren gemäß Anspruch 14, **dadurch gekennzeichnet, dass** genannte organische Base aus 4-Dimethylaminopyridin (DMAP), Pyridin, Picolin oder Lutidin ausgewählt wird.

18. Verfahren gemäß Anspruch 1, **dadurch gekennzeichnet, dass** in genanntem Schritt b) zu einer Lösung von genanntem Intermediat 2-Acyloxy-2-methyl-3-(4-fluorphenylthio)propionsäure Säurechlorid in der Gegenwart einer Base gegeben wird, um das korrespondierende gemischte Anhydrid zu ergeben, zu dem anschließend 4-Amino-2-trifluormethylbenzonitril gegeben wird, um das Intermediat N-[4-Cyano-3-(trifluormethyl)-phenyl]-3-[4-fluorphenylthio]-2-acyloxy-2-methylpropionami d zu ergeben.

19. Verfahren gemäß Anspruch 1, 14 und 18, **dadurch gekennzeichnet, dass** in genanntem Schritt b) genanntes Intermediat N-[4-Cyano-3-(trifluormethyl)-phenyl]-3-[4-fluorphenylthio]-2-acyloxy-2-methylpropionami d hydrolysiert wird, um genanntes N-[4-Cyano-3-(trifluormethyl)-phenyl]-3-[4-fluorphenylthio]-2-hydroxy-2-methyl-propiona mid zu ergeben.

20. Verfahren gemäß Anspruch 19, durch die Tatsache **gekennzeichnet**, dass in genanntem Schritt b) genannte Hydrolyse in Methanol durch die Zugabe von Kaliumcarbonat durchgeführt wird.

21. Verfahren gemäß Anspruch 1, **dadurch gekennzeichnet, dass** in genanntem Schritt a') und b) das Lösungsmittel Toluol ist und das Reaktionssystem eine Eintopfsynthese ist.

22. Verfahren gemäß Anspruch 1, **dadurch gekennzeichnet, dass** in genanntem Schritt c) genanntes N-[4-Cyano-3-(trifluormethyl)-phenyl]-3-[4-fluorphenylthio]-2-hydroxy-2-methylpropionamid in CH₂Cl₂ gelöst wird und mit m-Chlorperbenzoesäure zu Reaktion gebracht wird, um Bicalutamid zu ergeben.

23. Verfahren gemäß Anspruch 1, **dadurch gekennzeichnet, dass** eine Umkristallisierung von rohem Bicalutamid durchgeführt wird, um kristallisiertes Bicalutamid aus einem Kristallisationslösungsmittel ausgewählt aus Ethylacetat, Methylisobutylketon (MIBK), Acetonitril, Isopropylalkohol, Isobutylalkohol oder 1-Methoxy-2-propanol, gegebenenfalls mit Wasser zu ergeben.

24. Intermediat 2-Acyloxy-2-methyl-3-(4-fluorphenylthio)propionsäure, in welchem genannte Acyloxygruppe ausgewählt wird aus R1 = Methyl, Ethyl, Propyl, Butyl oder Phenyl.

25. Intermediat gemäß Anspruch 24, wobei genannte Acyloxygruppe Acetoxy ist.

26. Intermediat N-[4-Cyano-3-(trifluormethyl)-phenyl]-3-[4-fluorphenylthio]-2-acyloxy-2-methylpropionami d, in welchem genannte Acyloxygruppe ausgewählt wird aus R1 = Methyl, Ethyl, Propyl, Butyl oder Phenyl.

27. Intermediat gemäß Anspruch 26, wobei genannte Acyloxygruppe Acetoxy ist.

## Revendications

1. Procédé de synthèse de bicalutamide, à savoir le N-[4-cyano-3-(trifluorométhyl)-phényl]-3-[4-fluorophénylsulfonyl]-2-hydroxy-2-méthylpropionamide constitué par les étapes de :
a) réaction entre le 2-méthyl-oxirane-carboxylate d'alkyle et le 4-fluorothiophénol pour donner l'acide 2-hydroxy-2-méthyl-3-(4-fluorophénylthio)propionique,
b) réaction avec le 4-amino-2-trifluorométhyl-benzonitrile pour donner le N-[4-cyano-3-(trifluorométhyl)-phényl]-3-[4-fluorophénylthio]-2-hydroxy-2-méthylpropionamide,
c) oxydation dudit N-[4-cyano-3-(trifluorométhyl)-phényl]-3-[4-fluorophénylthio]-2-hydroxy-2-méthylpropionamide pour donner le bicalutamide,
**caractérisé en ce que** ledit acide 2-hydroxy-2-méthyl-3-(4-fluorophénylthio)propionique produit lors de l'étape a) subit, avant ladite étape b), une étape a') d'acylation du groupe hydroxyle en position 2 pour donner un intermédiaire acide 2-acyloxyl-2-méthyl-3-(4-fluoro-phénylthio)propionique qui, au cours de l'étape b), génère un intermédiaire successif N-[4-cyano-3-(trifluorométhyl)-phényl]-3-[4-fluorophénylthio]-2-acyloxy-2-méthylpropionamide.

2. Procédé selon la revendication 1, **caractérisé en ce que** lors de ladite étape a'), l'agent d'acylation est un anhydride (R1CO)₂O, R1 étant choisi parmi le méthyle, l'éthyle, le propyle, le n-butyle, le phényle.

3. Procédé selon la revendication 2, **caractérisé en ce que** lors de ladite étape a'), l'agent d'acylation est un anhydride acétique.

4. Procédé selon la revendication 1, **caractérisé en ce que** lors de ladite étape a), ledit 4-fluorothiophénol est dissous dans du méthanol anhydre, une solution de méthylate de sodium dans du méthanol est ajoutée, le méthanol est évaporé, le résidu est repris du toluène anhydre, ledit 2-méthyl-oxirane-carboxylate d'alkyle est ajouté à la solution, puis l'ester d'alkyle obtenu est hydrolysé dans un système biphasique toluène/hydroxyde de sodium dans l'eau.

5. Procédé selon la revendication 1, **caractérisé en ce que** lors de ladite étape a), l'eau est utilisée comme solvant en présence de carbonate ou de bicarbonate comme base.

6. Procédé selon la revendication 1, **caractérisé en ce que** lors de ladite étape a), l'eau est utilisée comme solvant en présence d'un alcali tel que la soude comme base.

7. Procédé selon la revendication 1, **caractérisé en ce que** lors de ladite étape a), le toluène est utilisé comme solvant en présence de méthylate de sodium comme base.

8. Procédé selon la revendication 1, **caractérisé en ce que** lors de ladite étape a), le 2-méthyl-oxirane-carboxylate d'alkyle et le 4-fluorothiophénol sont utilisés sans ajouter de solvant.

9. Procédé selon la revendication 8, **caractérisé en ce que** lors de ladite étape a), le 2-méthyl-oxirane-carboxylate d'alkyle et le 4-fluorothiophénol sont utilisés en présence de méthylate de sodium comme base.

10. Procédé selon les revendications 5, 6, 7, 9, **caractérisé en ce que** ladite base est en quantité catalytique.

11. Procédé selon la revendication 1, **caractérisé en ce que** lors de ladite étape a), le 2-méthyl-oxirane-carboxylate d'alkyle, dans lequel le groupe alkyle est Et, Pr, n-Bu, iso-Bu, est préparé par oxydation du 2-méthacrylate d'alkyle correspondant avec du MCPBA.

12. Procédé selon la revendication 1, **caractérisé en ce que** lors de ladite étape a), l'eau avec du bicarbonate de potassium est utilisée comme solvant pour le 2-méthyl-oxirane-carboxylate d'alkyle.

13. Procédé selon la revendication 1, **caractérisé en ce que** lors de ladite étape b), du chlorure de thionyle est ajouté à une solution dudit intermédiaire acide 2-acyloxyl-2-méthyl-3-(4-fluorophénylthio)propionique dans du toluène anhydre pour donner le chlorure d'acide correspondant.

14. Procédé selon la revendication 13, **caractérisé en ce que** lors de ladite étape b), on ajoute audit chlorure d'acide une base organique ou inorganique, puis le 4-amino-2-trifluorométhyl-benzonitrile pour donner l'intermédiaire N-[4-cyano-3-(trifluorométhyl)-phényl]-3-[4-fluorophénylthio]-2-acyloxy-2-méthylpropionamide.

15. Procédé selon la revendication 14, **caractérisé en ce que** lors de ladite étape b), on ajoute audit chlorure d'acide une base organique ou inorganique, puis le 4-amino-2-trifluorométhyl-benzonitrile, pour donner l'intermédiaire N-[4-cyano-3-(trifluorométhyl)-phényl]-3-[4-fluorophénylthio]-2-acyloxy-2-méthylpropionamide dans un solvant choisi parmi les hydrocarbures aromatiques et aliphatiques, es éthers, es cétones, es esters, es halogénoalcanes, avec une température de réaction maximale de 100°C.

16. Procédé selon la revendication 14, **caractérisé en ce que** lors de ladite étape b), on ajoute audit chlorure d'acide une base organique ou inorganique, puis le 4-amino-2-trifluorométhyl-benzonitrile pour donner l'intermédiaire N-[4-cyano-3-(trifluorométhyl)-phényl]-3-[4-fluorophénylthio]-2-acyloxy-2-méthylpropionamide dans un solvant choisi parmi les aprotiques polaires : DMA, DMF, NMP, DMI, acétonitrile avec une température de réaction maximale de 100°C.

17. Procédé selon la revendication 14, **caractérisé en ce que** ladite base organique est choisie parmi la 4-diméthylaminopyridine (DMAP), la pyridine, la picoline ou la lutidine.

18. Procédé selon la revendication 1, **caractérisé en ce que** lors de ladite étape b), on a ajouté à une solution dudit intermédiaire acide 2-acyloxy-2-méthyl-3-(4-fluorophénylthio)-propionique, du chlorure d'acyle en présence d'une base, pour donner l'anhydride mélangé correspondant, auquel le 4-amino-2-trifluorométhyl-benzonitrile est ensuite ajouté pour donner l'intermédiaire N-[4-cyano-3-(trifluorométhyl)-phényl]-3-[4-fluorophénylthio]-2-acyloxy-2-méthylpropionamide.

19. Procédé selon les revendications 1, 14 et 18, **caractérisé en ce que** lors de ladite étape b), ledit intermédiaire N-[4-cyano-3-(trifluorométhyl)-phényl]-3-[4-fluorophénylthio]-2-acyloxy-2-méthylpropionamide est hydrolysé pour donner ledit N-[4-cyano-3-(trifluorométhyl)-phényl]-3-[4-fluorophénylthio]-2-hydroxy-2-méthylpropionamide.

20. Procédé selon la revendication 19, **caractérisé par le fait que** lors de ladite étape b), ladite hydrolyse est réalisée dans du méthanol par l'addition de carbonate de potassium.

21. Procédé selon la revendication 1, **caractérisé en ce que** lors de ladite étape a') et de ladite étape b), le solvant est le toluène et les réactions sont effectuées en une seule fois.

22. Procédé selon la revendication 1, **caractérisé en ce que** lors de ladite étape c), ledit N-[4-cyano-3-(trifluorométhyl)-phényl]-3-[4-fluorophénylthio]-2-hydroxy-2-méthylpropionamide dissous dans du CH₂Cl₂ est mis à réagir avec de l'acide m-chloroperbenzoïque pour donner le bicalutamide.

23. Procédé selon la revendication 1, **caractérisé en ce qu'**une recristallisation de bicalutamide brut est réalisée pour donner du bicalutamide cristallisé à partir d'un solvant de cristallisation choisi parmi l'acétate d'éthyle, la méthyl isobutyl cétone (MIBK), l'acétonitrile, l'alcool isopropylique, l'alcool isobutylique ou le 1-méthoxy-2-propanol, éventuellement aqueux.

24. Intermédiaire acide 2-acyloxy-2-méthyl-3-(4-fluorophénylthio)propionique, dans lequel ledit groupe acyloxy est choisi parmi R1 = méthyle, éthyle, propyle, butyle ou phényle.

25. Intermédiaire selon la revendication 24, dans lequel ledit groupe acyloxy est l'acétoxy.

26. Intermédiaire N-[4-cyano-3-(trifluorométhyl)-phényl]-3-[4-fluorophénylthio]-2-acyloxy-2-méthylpropionamide, dans lequel ledit groupe acyloxy est choisi parmi R1 = méthyle, éthyle, propyle, butyle ou phényle.

27. Intermédiaire selon la revendication 26, dans lequel ledit groupe acyloxy est l'acétoxy.
